(19) Europäisches Patentamt
European Patent Office
Office européen des brevets

(11) Veröffentlichungsnummer: **0 001 571**
**B1**

(12) # EUROPÄISCHE PATENTSCHRIFT

(45) Veröffentlichungstag der Patentschrift: **08.04.81**

(21) Anmeldenummer: **78101023.6**

(22) Anmeldetag: **29.09.78**

(51) Int. Cl.³: **C 07 D 249/08, A 01 N 43/64**

(54) **Phenylazophenyloxy-triazolylverbindungen und Fungizide, die diese Verbindungen enthalten.**

(30) Priorität: **12.10.77 DE 2745827**

(43) Veröffentlichungstag der Anmeldung:
**02.05.79 Patentblatt 79/9**

(45) Bekanntmachung des Hinweises auf die Patenterteilung:
**08.04.81 Patentblatt 81/14**

(84) Benannte Vertragsstaaten:
**BE CH DE FR GB LU NL SE**

(56) Entgegenhaltungen:
**DE - A - 2 335 020**
**DE - A - 2 401 715**
**DE - A - 2 406 665**
**DE - A - 2 431 073**
**DE - A - 2 455 955**

(73) Patentinhaber: **BASF Aktiengesellschaft**
**Carl-Bosch-Strasse 38**
**D-6700 Ludwigshafen (DE)**

(72) Erfinder: **Rentzea, Costin, Dr.**
**Neuenheimer Landstrasse 72**
**D-6900 Heidelberg (DE)**
Erfinder: **Ammermann, Eberhard, Dr.**
**Sachsenstrasse 3**
**D-6700 Ludwigshafen (DE)**
Erfinder: **Pommer, Ernst-Heinrich, Dr.**
**Berliner Platz 7**
**D-6703 Limburgerhof (DE)**

**EP 0 001 571 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Phenylazophenyloxy-triazolylverbindungen und Fungizide, die diese Verbindungen enthalten

Die Erfindung betrifft wertvolle substituierte Phenylazophenyloxy-triazolyl-O,N-acetate, deren Salze und Metallkomplexverbindungen mit guter fungizider Wirkung, Fungizide, die diese Verbindungen enthalten und Verfahren zur Bekämpfung von Pilzen mit diesen Verbindungen.

Es ist bekannt, daß das 1 - [2 - (2,4 - Dichlorphenyl) - 2 - (2 - propenyloxy) - äthyl] - 1H - imidazol eine fungizide Wirksamkeit hat (vgl. DE—OS 20 63 857). Seine Wirkung ist insbesondere gegenüber Mehltaupilzen und Rostpilzen unzureichend. Aus diesem Grunde ist es für den Gebrauch als Pflanzenschutzmittel zur Bekämpfung von Pilzen wenig geeignet.

Es wurde gefunden, daß die Phenylazophenyloxy-triazolyl-O,N-acetale der allgemeinen Formel I

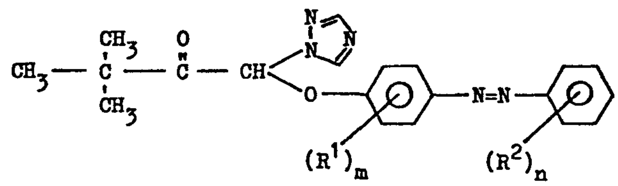

(I)

in der $R^1$ und $R^2$ gleich oder verschieden sind und Alkyl, Alkoxy, Alkylsulfoxy mit jeweils bis zu 4 C-Atomen, Halogen (Fluor, Chlor, Brom und Jod), Trifluormethyl oder Nitro bedeuten und m eine Zahl von 0, 1, 2, 3 und 4 und n eine Zahl von 0, 1, 2, 3, 4 oder 5 sein kann, sowie deren Salze und Metallkomplexverbindungen eine sehr gute fungizide Wirksamkeit bein ausgezeichneter Pflanzenverträglichkeit zeigen. Salze sind z.B. Hydrochloride, Hydrobromide, Sulfate, Oxalate, Dodecylbenzolsulfonate oder Nitrate.

Die Metallkomplexverbindungen haben die allgemeine Formel II

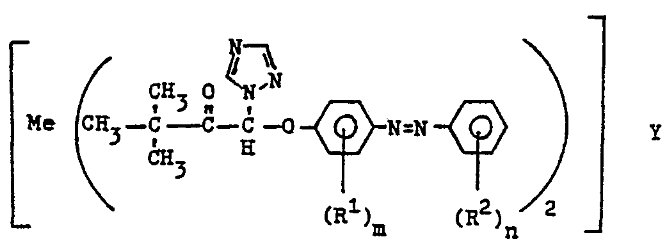

(II)

worin Me für ein Metallkation der Metalle Kupfer, Zink, Zinn, Mangan, Eisen, Cobalt oder Nickel steht und Y ein dem Metall-Kation äquivalentes Anion einer anorganischen Säure, z.B. Salzsäure, Schwefelsäure, Phosphorsäure, Salpetersäure oder Bromwasserstoffsäure bedeutet.

Es wurde weiterhin gefunden, daß man die Phenylazophenloxy - (1,2,4 - triazolyl - 1 - ) - O,N - acetale der allgemeinen Formel I erhält, wenn man ein 1 - (Phenylazophenyloxy) - 1 - halogen - 3,3 - dimethylbutan - 2 - cn der allgemeinen Formel III mit 1,2,4-Triazol in einem Lösungsmittel in Gegenwart eines basischen Katalysators umsetzt.

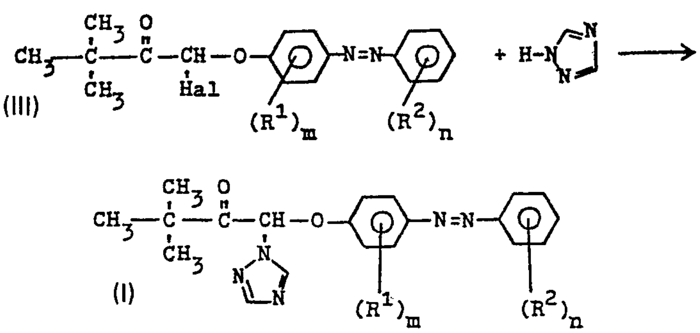

(III)

(I)

In den allgemeinen Formeln I und III haben $R^1$, $R^2$, m und n die oben genannten Bedeutungen.

Die Salze werden hergestellt durch Umsetzung einer Verbindung der allgemeinen Formel I mit einer Säure, gegebenenfalls in einem Lösungsmittel.

Die Metal komplexverbindungen der allgemeinen Formel II erhält man, wenn man die 1 - (Arylazoaryloxy) - 1 - (1,2,4 - triazolyl - 1 - ) - N,O - acetale der allgemeinen Formel I mit Metallsalzen der allgemeinen Formel IV

MeY (IV)

in welcher Me und Y die oben erwähnte Bedeutung haben, in Gegenwart eines Lösungsmittels umsetzt.

Die als Ausgangsstoffe verwendeten Verbindungen der allgemeinen Formel III können durch Halogenierung der nach bekannten Verfahren hergestellten 1 - Phenylazophenyloxy - 3,3 - dimethylbutan - 2 - one der allgemeinen Formel V erhalten werden.

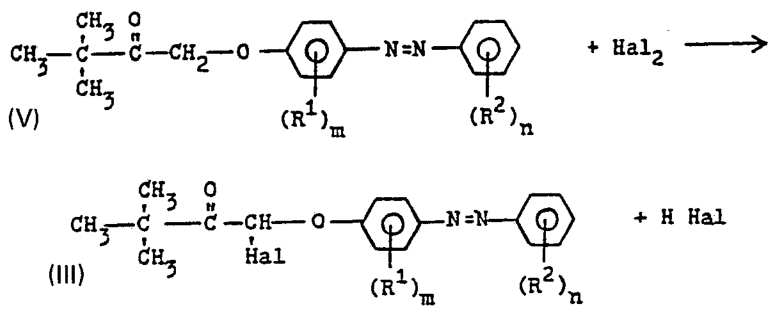

(V)
(III)

In diesen allgemeinen Formeln besitzen $R^1$, $R^2$, m und n die zuvor erläuterten Bedeutungen und Hal bedeutet ein Halogenatom. Als Halogenierungsmittel können beispielsweise Brom, N-Bromsuccinimid, Sulfurylchlorid oder N-Chlorsuccinimid verwendet werden. Die Halogenierung wird zweckmäßig in einem inerten Lösungsmittel wie $CH_2Cl_2$, $CHCl_3$, $CCl_4$ oder 1,2-Dichloräthan durchgeführt.

Für die Umsetzung mit Triazol zur Herstellung der erfindungsgemäßen 1 - Arylazoaryloxy - 1 - (triazolyl) - N,O - acetale der allgemeinen Formel I sind beispielsweise Aceton, Methyläthylketon und Acetonitril vorteilhafte Lösungsmittel. Als basische Katalysatoren können wasserfreie anorganische Salze, wie $K_2CO_3$ und $Na_2CO_3$ oder organische Basen, wie Pyridin, Triäthylamin oder N,N-Dimethylcyclohexylamin eingesetzt werden. Die Reaktion wird am besten bei der Siedetemperatur des verwendeten Lösungsmittels durchgeführt.

Fur die Herstellung der Metallkomplexe der allgemeinen Formel II kommen alle mit Wasser mischbaren Lösungsmittel in Frage. Gut geeignet sind Methanol, Äthanol und Tetrahydrofuran.

Man arbeitet meist zwischen 10 und 50°C.

Als Beispiele für die 1 - (4 - Phenylazophenlyoxy) - 1 - (1,2,4 - triazol - (1) - ) - 3,3 - dimethylbutan - 2 - on Derivate seien im einzelnen genannt:

1-(1,2,4-Triazolyl-(1))-1-(4'-phenylazo)-phenoxy-3,3-dimethylbutan-2-on
1-(1,2,4-Triazolyl-(1))-1-(4'-(2''-fluorphenylazo)-phenoxy-3,3-dimethylbutan-2-on
1-(1,2,4-Triazolyl-(1))-1-(4'-(3''-fluorphenylazo)-phenoxy)-3,3-dimethylbutan-2-on
1-(1,2,4-Triazolyl-(1))-1-(4'-(4''-fluorphenylazo)-phenoxy)-3,3-dimethylbutan-2-on
1-(1,2,4-Triazolyl-(1))-1-(4'-(4''-chlorphenylazo)-phenoxy)-3,3-dimethylbutan-2-on
1-(1,2,4-Triazolyl-(1))-1-(4'-(4''-bromophenylazo)-phenoxy)-3,3-dimethylbutan-2-on
1-(1,2,4-Triazolyl-(1))-1-(4'-(2'',4''-dichlorphenylazo)-phenoxy)-3,3-dimethylbutan-2-on
1-(1,2,4-Triazolyl-(1))-1-(4'-(3'',4''-dichlorphenylazo)-phenoxy)-3,3-dimethylbutan-2-on
1-(1,2,4-Triazolyl-(1))-1-(4'-(2'',4'',5''-trichlorphenylazo)-phenoxy)-3,3-dimethylbutan-2-on
1-(1,2,4-Triazolyl-(1))-1-(4'-(2'',4''-difluorphenylazo)-phenoxy)-3,3-dimethylbutan-2-on
1-(1,2,4-Triazolyl-(1))-1-(4'-(3'',4''-difluorphenylazo)-phenoxy)-3,3-dimethylbutan-2-on
1-(1,2,4-Triazolyl-(1))-1-(4'-(3''-chlor-4''-fluorphenylazo)-phenoxy)-3,3-dimethylbutan-2-on
1-(1,2,4-Triazolyl-(1))-1-(4'-(3''-trifluormethylphenylazo)-phenoxy)-3,3-dimethylbutan-2-on
1-(1,2,4-Triazolyl-(1))-1-(4'-(2''-methyl-5''-chlorphenylazo)-phenoxy)-3,3-dimethylbutan-2-on
1-(1,2,4-Triazolyl-(1))-1-(4'-(2''-chlor-4''-methylsulfoxy-phenylazo)-phenoxy)-3,3-dimethylbutan-2-on
1-(1,2,4-Triazolyl-(1))-1-(4'-(3''-chlor-4''-methoxyphenylazo)-phenoxy)-3,3-dimethylbutan-2-on
1-(1,2,4-Triazolyl-(1))-1-(4'-(3''-chlor-4''-methoxyphenylazo)-phenoxy)-3,3-dimethylbutan-2-on
1-(1,2,4-Triazolyl-(1))-1-(2'-chlor-4'-(phenylazo)-phenoxy)-3,3-dimethylbutan-2-on
1-(1,2,4-Triazolyl-(1))-1-(2'-chlor-4'-(4''-fluorphenylazo)-phenoxy)-3,3-dimethylbutan-2-on
1-(1,2,4-Triazolyl-(1))-1-(2'-chlor-4'-(4''-chlorphenylazo)-phenoxy)-3,3-dimethylbutan-2-on
1-(1,2,4-Triazolyl-(1))-1-(2'-chlor-4'-(2'',4''-dichlorphenylazo)-phenoxy)-3,3-dimethylbutan-2-on

Als Beispiele für die Metallkomplexe seien im einzelnen gennant:

Bis - (1 - (1,2,4 - Triazolyl - (1)) - 1 - (4' - (2'' - fluorphenylazo) - phenoxy) - 3,3 - dimethylbutan - 2-on) - kupfer - II - chlorid
Bis - (1 - (1,2,4 - Triazolyl - (1)) - 1 - (4' - (3'' - fluorphenylazo) - phenoxy) - 3,3 - dimethylbutan - 2-on) - kupfer - II - chlorid
Bis - (1 - (1,2,4 - Triazolyl - (1)) - 1 - (4' - (4'' - fluorphenylazo) - phenoxy) - 3,3 - dimethylbutan - 2-on) - kupfer - II - chlorid

Bis - (1 - (1,2,4 - Triazolyl - (1)) - 1 - (4' - (4'' - chlorphenylazo) - phenoxy) - 3,3 - dimethylbutan - 2-on) - kupfer - II - chlorid

Bis - (1 - (1,2,4 - Triazolyl - (1)) - 1 - (4' - (2'',4'' - difluorphenylazo) - phenoxy) - 3,3 - dimethylbutan-2 - on) - kupfer - II - chlorid

Bis - (1 - (1,2,4 - Triazolyl - (1)) - 1 - (2' chlor - 4' - (4'' - fluorphenylazo) - phenox) - 3,3 - dimethylbutan - 2 - on) - kupfer - II - chlorid

Bis - (1 - (1,2,4 - Triazolyl - (1)) - 1 - (4' - (4'' - fluorphenylazo) - phenoxy) - 3,3 - dimethylbutan - 2-on) - zink - II - chlorid

Bis - (1 - (1,2,4 - Triazolyl - (1)) - 1 - (4' - (2'',4'' - dichlorphenylazo) - phenoxy) - 3,3 - dimethylbutan-2 - on) - zink - II - chlorid

Bis - (1 - (1,2,4 - Triazolyl - (1)) - 1 - (4' - (3'' - chlor - 4'' - fluorphenylazo) - phenoxy) - 3,3 - dimethylbutan - 2 - on) - zink - II - chlorid

Bis - (1 - (1,2,4 - Triazolyl - (1)) - 1- (2' - chlor - 4' - (4'' - fluorphenylazo) - phenoxy) - 3,3 - dimethylbutan - 2 - on) - zink - II - chlorid

Beispiel 1

A. Umsetzung von

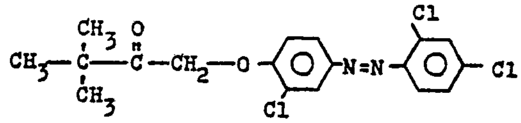

mit N-Bromsuccinimid zu

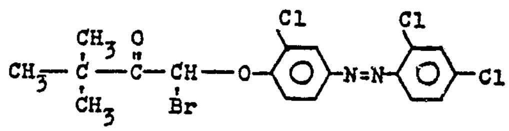

70 g 1 - (2' - Chlor - 4' - (2'',4'' - dichlorphenylazo) - phenoxy) - 3,3 - dimethylbutan - 2 - on, 31,7 g N-Bromsuccinimid und 1 ml Brom werden in 500 ml Tetrachlorkohlenstoff für 5 Stunden bei 70°C gerührt. Nach dem Abkühlen wird filtriert und das Filtrat eingeengt. Der Rückstand wird mit n-Pentan gewaschen.

Ausbeute: 79,5 g (94% der theor. Ausbeute)
Fp. 119 bis 123°C.

Umsetzung von

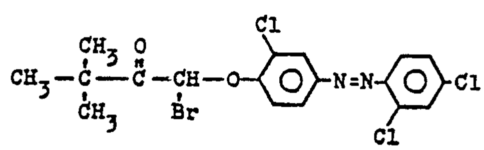

mit 1,2,4-Triazol zu

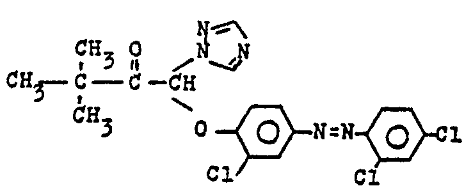

53 g 1 - (2' - Chlor - 4' - (2'',4'' - dichlorphenylazo) - phenoxy) - 1 - brom - 3,3 - dimethylbutan - 2 - on, 7,7 g 1,2,4-Triazol- und 28 g Kaliumcarbonat werden in 300 ml Aceton für 12 Stunden unter Sieden und Rückfluß gerührt. Nach dem Abkühlen wurde abfiltriert, das Filtrat eingeengt und der Rückstand säulenchromatographisch an Kieselgel aufgetrennt. Elutionsmittel waren Cyclohexan und Essigester; man erhält hellgelbe Kristalle der Verbindung 1 - (1,2,4 - Triazolyl - (1)) - 1 - (2' - chlor - 4' - (2'',4'' - dichlorphenylazo) - phenoxy) - 3,3 - dimethyl - butan - 2 - on (Verbindung Nr. 20).

Ausbeute: 19 g (37% der theoret. Ausbeute)
Fp. 168°C

Entsprechend wurden die in Tabelle 1 aufgeführten Verbindungen hergestellt.

Tabelle 1

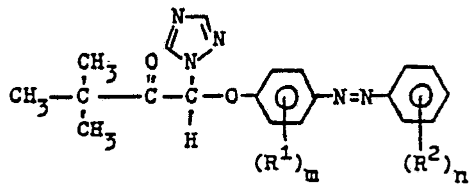

| Verbindung Nr. | $R^1$ | m | $R^2$ | n | Schmelzpunkt °C |
|---|---|---|---|---|---|
| 1 | — | 0 | — | 0 | 89—91 |
| 2 | — | 0 | 2-F | 1 | 71—75 |
| 3 | — | 0 | 3-F | 1 | 101—104 |
| 4 | — | 0 | 4-F | 1 | 104—105 |
| 5 | — | 0 | 4-Cl | 1 | 104—106 |
| 6 | — | 0 | 4-Br | 1 | 122—125 |
| 7 | — | 0 | 2,4-$Cl_2$ | 2 | 122—124 |
| 8 | — | 0 | 3,4-$Cl_2$ | 2 | 122—125 |
| 9 | — | 0 | 2,4,5-$Cl_3$ | 3 | 117—118 |
| 10 | — | 0 | 2,4-$F_2$ | 2 | 105—106 |
| 11 | — | 0 | 3,4-$F_2$ | 2 | 86—87 |
| 12 | — | 0 | 3-Cl, 4-F | 2 | 116—120 |
| 13 | — | 0 | 3-$CF_3$ | 1 | 104—106 |
| 14 | — | 0 | 2-$CH_3$, 5-Cl | 2 | 102—108 |
| 15 | — | 0 | 2-Cl, 4-$SO_2CH_3$ | 2 | 143—147 |
| 16 | — | 0 | 3-Cl, 4-$OCH_3$ | 2 | 132—135 |
| 17 | 2-Cl | 1 | — | 0 | 108—110 |

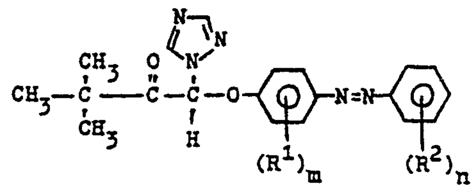

| Verbindung Nr. | $R^1$ | m | $R^2$ | n | Schmelzpunkt °C |
|---|---|---|---|---|---|
| 18 | 2-Cl | 1 | 4-F | 1 | 105—107 |
| 19 | 2-Cl | 1 | 4-Cl | 1 | 134—136 |
| 20 | 2-Cl | 1 | 2,4-$Cl_2$ | 2 | 168 |
| 31 | 2-F | 1 | — | 0 | 110—112 |
| 32 | 2-F | 1 | 4-F | 1 | 90—92 |
| 33 | 2-Br | 1 | — | 0 | 117—119 |
| 34 | 2-Br | 1 | 4-F | 1 | 91—93 |

Beispiel 2

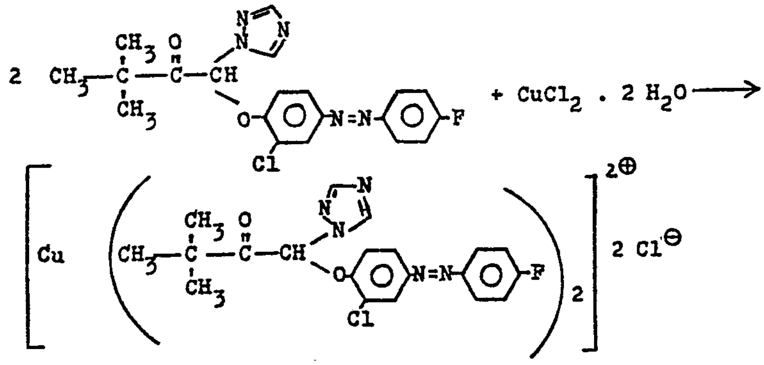

10 g 1 - (1,2,4 - Triazolyl - (1)) - 1 - (2′ - chlor - 4′ - (4″ - fluorphenylazo) - phenoxy) - 3,3 - dimethylbutan - 2 - on (Verbindung Nr. 18) werden bei 40°C in 200 ml Äthanol gelöst und mit einer Lösung von 8,5 g Kupferchloriddihydrat in 100 ml Äthanol tropfenweise versetzt. Nach Stehen über Nacht werden die entsandenen hellgrünen Kristalle abgesaugt (Verbindung Nr. 26).

Ausbeute: 7 g
Fp. 102 bis 104°C.

Beispiel 3

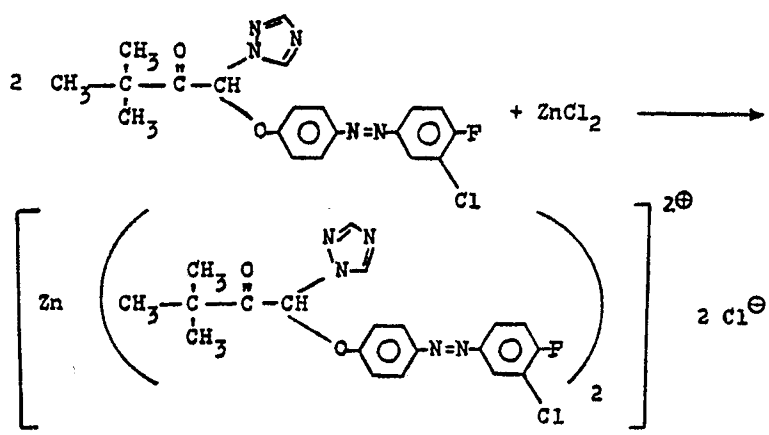

Tabelle 2

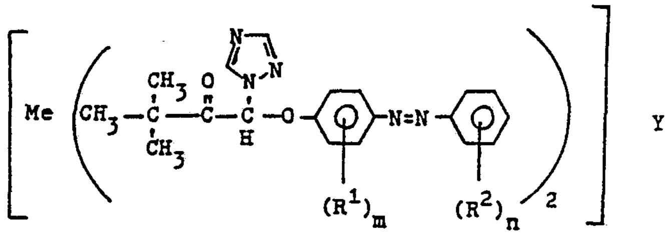

| Verbindung Nr. | Me | $R^1$ | m | $R^2$ | n | Y | Fp.°C |
|---|---|---|---|---|---|---|---|
| 21 | Cu | — | 0 | 2-F | 1 | Cl | 105—110 |
| 22 | Cu | — | 0 | 3-F | 1 | Cl | 140—142 |
| 23 | Cu | — | 0 | 4-F | 1 | Cl | 103—105 |
| 24 | Cu | — | 0 | 4-Cl | 1 | Cl | 124—127 |
| 25 | Cu | — | 0 | 2,4-$F_2$ | 2 | Cl | 139—141 |
| 26 | Cu | 2-Cl | 1 | 4-F | 1 | Cl | 102—104 |
| 27 | Zn | — | 0 | 4-F | 1 | Cl | 168—171 |
| 28 | Zn | — | 0 | 2,4-$Cl_2$ | 2 | Cl | 174—176 |
| 29 | Zn | — | 0 | 3-Cl, 4-F | 2 | Cl | 173—178 |
| 30 | Zn | 2-Cl | 1 | 4-F | 1 | Cl | 140—147 |

Die erfindungsgemäßen Verbindungen und ihre Salze und Metallkomplexverbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide, besonders aber auch als Beizmittel eingesetzt werden.

Besonders interessant sind die fungiziden Verbindungen für die Bekämpfung einer Vielzahl von Pilzen an verschiedenen Kulturpflanzen oder ihren Samen.

Unter Kulturpflanzen verstehen wir in diesem Zusammenhang insbesondere Weizen, Roggen, Gerste, Hafer, Reis, Mais, Baumwolle, Soja, Kaffee, Zuckerrohr, Obst und Zierpflanzen im Gartenbau sowie Gemüse wie Gurken, Bohnen und Kürbisgewächse.

Die erfindungsgemäßen Verbindungen sind insbesondere geeignet zur Bekämfung folgender Pflanzenkrankheiten:

Erysiphe graminis (echter Mehltau) in Getreide,
Erysiphe cichoriacearum (echter Mehltau) an Kürbisgewächsen
Podosphaera leucotricha an Äpfeln,
Uncinula necator an Reben,
Erysiphe polygoni an Bohnen,
Sphaerotheca pannosa an Rosen,
Puccinia-Arten an Getreide,
Rhizoctonia solani an Baumwolle sowie
Helminthosphoriumarten an Getreide,
Ustilago-Arten an Getreide und Zuckerrohr,
Rhynchosporium secale an Getreide.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die erfindungsgemäßen Wirkstoffe können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Äthanolamin, Dimethylformamid) und Wasser; Trägerstoffe wie natürlich Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionsiche Emulgatoren (z.B. Polyoxyäthylen - Fettalkohol - Äther, Alkylsulfonate und Arylsulfonate) und Dispergiermittel, wie Lignin-, Sulfitablaugen und Methylcellulose.

Die fungiziden Mittel enthalten im allgemeinen zwischen 0,1 und 95 Gewichtsprozent Wirkstoff, vorzugsweise zwischen 0,5 und 90.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Die Aufwandmengen liegen nach Art des gewünschten Effektes zwischen 0,01 und 3, vorzugsweise jedoch zwischen 0,01 und 1 kg Wirkstoff pro Hektar.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Metallkomplexen kombiniert werden können, sind beispielsweise:

Dithiocarbamate und deren Derivate, wie
Ferridimethyldithiocarbamat,
Zinkdimethyldithiocarbamat,
Manganäthylenbisdithiocarbamat,
Mangan-Zink-äthylendiamin-bis-dithiocarbamat,
Zinkäthylenbisdithiocarbamat,
Tetramethylthiuramdisulfide,
Ammoniak-Komplex von Zink-(N,N-äthylen-bis-dithiocarbamat) und
N,N'-Polyäthylen-bis-(thiocarbamoyl)-disulfid,
Zink-(N,N'-propylen-bis-dithiocarbamat,
Ammoniak-Komplex von Zink-(N,N'-propylen-bis-dithiocarbamat) und
N,N'-Polypropylen-bis-(thiocarbamoyl)-disulfid.

Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;

heterocyclische Strukturen, wie
N-Trichlormethylthio-tetrahydrophthalimid,
N-Trichlormethylthio-phthalimid,
2-Heptadecyl-2-imidazolin-acetat,
2,4-Dichlor-6-(o-chloranilino)-s-triazin,
O,O-Diäthyl-phthalimidophosphonthioat,
5-Amino-1-(bis-(dimethylamino)-phosphinyl)-3-phenyl-1,2,4-triazol,
5-Äthoxy-3-trichlormethyl-1,2,4-thiadiazol,
2,3-Dicyano-1,4-dithiaanthrachinon,
2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-Rhodanmethylthio-benzthiazol,
4-(2-Chlorphenylhydrozono)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin-4,4-dioxid,

2,3-Dihydro-5-carboxanilido-6-methyl-1,4-oxathiin,
2-(Furyl-(2))-benzimidazol,
Piperazin-1,4-diyl-bis-(1-(2,2,2-trichlor-äthyl)-formamid),
2-(Thiazolyl-(4))-benzimidazol,
5-Butyl-2-dimethylamino-4-hydroxy-6-methyl-pyrimidin,
Bis-(p-Chlorphenyl)-3-pyridinmethanol,
1,2-Bis-(3-äthoxycarbonyl-2-thioureido)-benzol,
1,2-Bis-(3-methoxycarbonyl-2-thioureido)-benzol und

verschiedene Fungizide, wie
Dodecylguanidinacetat,
3-(3-(3,5-Dimethyl-2-oxycyclohexyl)-2-hydroxyäthyl)-glutarimid,
Hexachlorbenzol,
N-Dichlorfluormethylthio-N',N'-dimethyl-N-phenyl-schwefelsäurediamid,
2,5-Dimethyl-furan-3-carbonsäureanilid,
2,5-Dimethyl-furan-3-carbonsäure-cyclohexylamid,
2-Methyl-benzoesäure-anilid,
2-Jod-benzoesäure-anilid,
1-(3,4-Dichloranilino)-1-formylamino-2,2,2-trichloräthan,
2,6-Dimethyl-N-tridecyl-morpholin bzw. dessen Salze,
2,6-Dimethyl-N-cyclododecyl-morpholin bzw. dessen Salze.

Beispiel 4

Wirkung gagen Weizenmehltau

Blätter von in Töpfen gewachsenen Weizenkeimlingen der Sorte "Jubilar" werden mit wäßrigen Emulsionen aus 80% (Gewichtsprozent) Wirkstoff und 20% Emulgiermittel besprüht und nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temeraturen zwischen 20 und 22°C und 75 bis 80% relativer Luftfeuchtigkeit aufgestellt. Nach 10 Tagen wird das Ausmaß der Mehltauentwicklung ermittelt.

| Wirkstoff Nr. | Befall der Blätter nach Spritzung mit .. %iger Wirkstoffbrühe | | |
|---|---|---|---|
| | 0,05 | 0,025 | 0,012 |
| 2 | 0 | 0 | 0 |
| 4 | 0 | 0 | 1 |
| 5 | 0 | 0 | 1 |
| 10 | 0 | 0 | 0 |
| 11 | 0 | 0 | 0 |
| 12 | 0 | 1 | 2 |
| 15 | 0 | 2 | 2—3 |
| 18 | 0 | 0 | 2 |
| 20 | 0 | 1 | 2 |
| 21 | 0 | 0 | 3 |
| 23 | 0 | 0 | 2 |
| 25 | 0 | 1 | 1 |
| 27 | 0 | 0 | 2 |
| 28 | 0 | 1 | 1—2 |
| 29 | 0 | 1 | 2—3 |
| Vergleichswirkstoffe | | | |
| 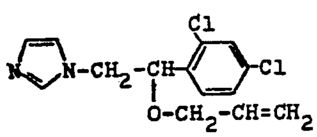 | 2 | 3 | 3 |
| bekannt aus DE—OS 20 63 857 | | | |
| 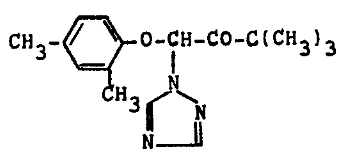 | 1 | 2 | 2—3 |
| bekannt aus DE—OS 24 01 715 | | | |
| Kontrolle (unbehandelt) | | 5 | |

O=kein Pilzbefall, abgestuft bis 5=Totalbefall

Beispiel 5

Wirkung gegen Weizenbraunrost

Blätter von in Töpfen gewachsenen Weizensämlingen der Sorte "Caibo" werden mit Sporen des Braunrostes (Puccinia recondita) bestäubt. Danach werden die Töpfe für 24 Stunden bei 20 bis 22°C in eine Kammer mit hoher Luftfeuchtigkeit (90 bis 95%) gestellt. Während dieser Zeit keimen die Sporen aus und die Keimschläuche dringen in das Blattgewebe ein. Die infizierten Pflanzen werden anschließend mit 0,05, 0,025 und 0,012%igen (Gew.%) wäßrigen Spritzbrühen, die 80% Wirkstoff und 20% Ligninsulfonat in der Trockensubstanz enthalten, tropfnaß gespritzt. Nach dem Antrocknen des Spritzbelages werden die Versuchspflanzen im Gewächshaus bei Temperaturen zwischen 20 und 22°C und 65 bis 70% relativer Luftfeuchte aufgestellt. Nach 8 Tagen wird das Ausmaß der Rostpilzentwicklung auf den Blättern ermittelt.

| Wirkstoff Nr. | Befall der Blätter nach Spritzung mit ..%iger Wirkstoffbrühe | | |
|---|---|---|---|
| | 0,05 | 0,025 | 0,012 |
| 2 | 0 | 0 | 0 |
| 4 | 0 | 0 | 0 |
| 10 | 0 | 0 | 0 |
| 11 | 0 | 0 | 1 |
| 18 | 0 | 0 | 0 |
| 21 | 0 | 0 | 2 |
| 23 | 0 | 0 | 0 |
| 25 | 0 | 0 | 0 |
| 26 | 0 | 0 | 0 |
| 27 | 0 | 0 | 1 |
| 30 | 0 | 0 | 0 |
| Vergleichswirkstoffe | | | |
| 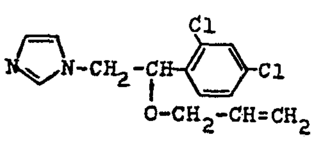 bekannt aus DE—OS 20 63 857 | 3 | 4 | 5 |
| 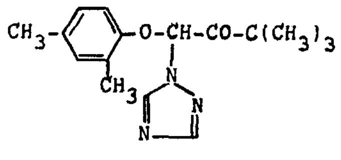 bekannt aus DE—OS 24 01 715 | 4 | 4 | 4 |
| Kontrolle (unbehandelt) | | 5 | |

0=kein Pilzbefall, abgestuft bis 5=Totalbefall

Beispiel 6

Wirkung gegen Gerstenmehltau

Blätter von in Töpfen gewachsenen Gerstenkeimlingen werden mit wäßrigen Emulsionen aus, 80% (Wirkstoff und 20% Emulgiermittel besprüht und nach dem Antrocknen des Spritzbelages mit Oidien (Sporen) des Gerstenmehltaus (Erysiphe graminis var. hordei) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff Nr. | Befall der Blätter nach Spritzung mit %iger Wirkstoffbrühe | | |
|---|---|---|---|
| | 0,05 | 0,025 | 0,012 |
| 2 | 0 | 0 | 0 |
| 4 | 0 | 0 | 1 |
| 5 | 0 | 0 | 0 |
| 10 | 0 | 0 | 0 |
| 11 | 0 | 0 | 1 |
| 12 | 0 | 2 | 3 |
| 18 | 0 | 0 | 2 |
| 19 | 0 | 0 | 0 |
| 20 | 0 | 0 | 0 |
| 21 | 0 | 1 | 3 |
| 23 | 0 | 0 | 0 |
| 24 | 0 | 2 | 2—3 |
| 25 | 0 | 0 | 2 |
| 26 | 0 | 1 | 2 |
| 27 | 0 | 1 | 2 |
| 28 | 0 | 0 | 0 |
| 29 | 0 | 1 | 2 |
| 30 | 0 | 1 | 2 |
| Kontrolle (unbehandelt) | | 5 | |

0=kein Pilzbefall, abgestuft bis 5=Totalbefall

Beispiel 7

Man vermischt 90 Gewichtsteile der Verbindung 1 mit 10 Gewichtsteilen N-Methyl-$\alpha$-pyrrolidon und erhält eine Lösung, die zur Anwendung in Form kleinster Tropfen geeignet ist.

Beispiel 8

20 Gewichtsteile der Verbindung 2 werden in einer Mischung gelöst, die aus 80 Gewichtsteilen Xylol, 10 Gewichtsteilen des Anlargerungsproduktes von 8 bis 10 Mol Äthylenoxid an 1 Mol Ölsäure-N-monoäthanolamid, 5 Gewichtsteilen Calciumsalz der Dodecylbenzolsulfonsäure und 5 Gewichtsteilen des Anlagerungsproduktes von 40 Mol Äthylenoxid an 1 Mol Ricinusöl besteht. Durch Ausgießen und feines Verteilen der Lösung in 10 000 Gewichsteilen Wasser erhält man eine wäßrige Dispersion, die 0,02 Gewichtsprozent des Wirkstoffs enthält.

Beispiel 9

20 Gewichtsteile der Verbindung 3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man ein wäßrige Dispersion, die 0,02 Gewichtsprozent des Wirkstoffs enthält.

Beispiel 10

20 Gewichtsteile der Verbindung 1 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 11

20 Gewichtsteile des Wirkstoffs werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen puverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

Beispiel 12

3 Gewichtsteile der Verbindung 3 werden mit 97 Gewichtsteilen feinteiligem Kaolin innig vermischt. Man erhält auf diese Weise eine Stäubemittel, das 3 Gewichtsprozent des Wirkstoffs enthält.

Beispiel 13

30 Gewichtsteile der Verbindung 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 14

40 Gewichtsteile des Wirkstoffs 1 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gewichtsprozent Wirkstoff enthält.

Beispiel 15

20 Teile des Wirkstoffs 2 werden mit 2 Teilen Calciumsalz der Dodecylbenzolsulfonsäure, 8 Teilen Fettalkohol-polyglykoläther, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Beispiel 16

Gurkenmehltau

Blätter von in Töpfen gewachsenen Gurkenkeimlingen werden mit wäßrigen Emulsionen aus 80% Wirkstoff und 20% Emulgiermittel besprüht und nach dem Antrocknen des Spritzbelages mit Oidien (Sporen) des Gurkenmehltaus (Erysiphe cichoriacearum) bestäubt. Die Versuchspflanzen werden anschließend im Gewächshaus bei Temperaturen zwischen 20 und 22°C und 75 bis 80% relativer Luftfeuchtigkeit aufgestellt. Nach 10 Tagen wird das Ausmaß der Mehltaupilzentwicklung ermittelt.

| Wirkstoff | Befall der Blätter nach Spritzung mit ...%iger Wirkstoffbrühe 0,05 | 0,025 | 0,0125 |
|---|---|---|---|
| 3 | 0 | 0 | 0 |
| Kontrolle (unbehandelt) | 5 | | |

0 kein Befall, abgestuft bis 5 Totalbefall

**Patentansprüche**

1. 1 - (1,2,4 - Triazolyl - (1) - ) - 1 - (4' - phenylazo) - phenyloxy - 3,3 - dimethylbutan - 2 - on - Verbindungen der allgemeinen Formel I

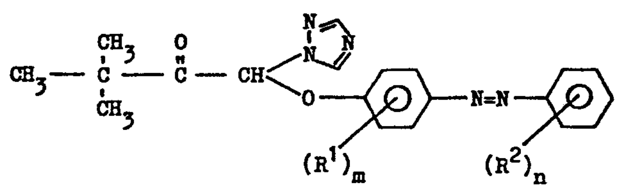

(I)

in der

$R^1$ und $R^2$ gleich oder verschieden sind und Alkyl, Alkoxy, Alkylsulfoxy mit jeweils bis zu 4 C-Atomen, Halogen, Trifluormethyl und Nitro bedeuten und

m eine Zahl von 0, 1, 2, 3 und 4 und

n eine Zahl von 0, 1, 2, 3, 4 und 5 sein kann sowie deren Salze und Metallkomplexverbindungen der Metalle Cu, Zn, Sn, Mn, Fe, Co und Ni.

2. Fungizid, enthaltend eine 1 - (1,2,4 - Triazolyl - (1) - ) - 1 - (4' - phenylazo) - phenyloxy - 3,3 - dimethylbutan - 2 - on - Verbindung gemäß Anspruch 1.

3. Verfahren zur Herstellung eines Fungizids, dadurch gekennzeichnet, daß man einen festen oder flüssigen Trägerstoff vermischt mit einer 1 - (1,2,4 - Triazolyl - (1) - ) - 1 - (4' - phenylazo) - phenyloxy - 3,3 - dimethylbutan - 2 - on - Verbindung gemäß Anspruch 1.

4. 1 - (1,2,4 - Triazol - (1) - ) - 1 - (4' - phenylazo) - phenyloxy - 3,3 - dimethylbutan - 2 - on - Verbindung, ausgewählt aus der Gruppe bestehend aus

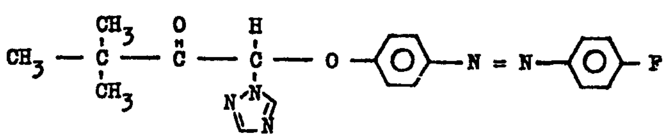

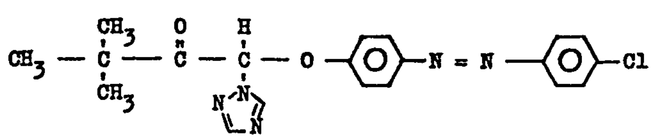

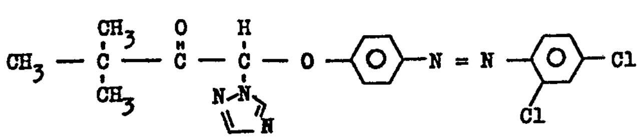

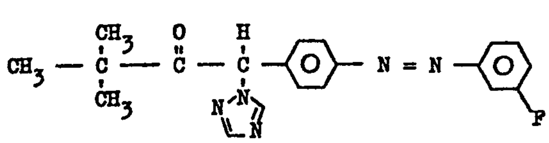

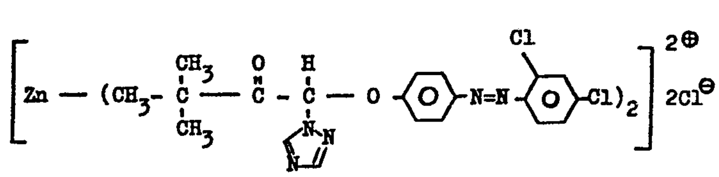

**Revendications**

1. 1 - (1,2,4 - triazolyl - (1) - ) - 1 - (4' - phénylazo) - phényloxy - 3,3 - diméthylbutane - 2 - ones de formule générale I

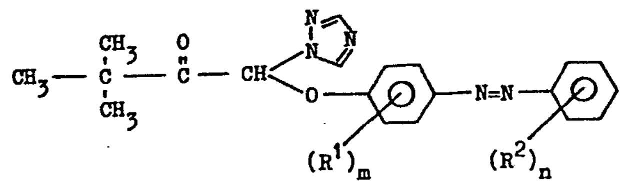

(I)

dans laquelle

$R^1$ et $R^2$ sont identiques ou différents et représentent des restes alkyle, alcoxy, alkylsulfoxy portant, dans chaque cas, jusqu'à 4 atomes de carbone, un halogène, un reste trifluorométhyle ou un groupe nitro, *m* est un des nombres 0, 1, 2, 3 et 4 et *n* est un des nombres 0, 1, 2, 3, 4 et 5, ainsi que leurs sels et leurs complexes métalliques avec les métaux Cu, Zn, Sn, Mn, Fe, Co et Ni.

2. Fongicide contenant une 1 - (1,2,4 - triazolyl - (1) - ) - 1 - (4' - phénylazo) - phényloxy - 3,3 - diméthylbutane - 2 - one selon la revendication 1.

3. Procédé de préparation d'un fongicide, caractérisé par le fait qu'on mélange un support solide ou liquide avec une 1 - (1,2,4 - triazolyl - 1 - ) - 1 - (4' - phénylazo) - phényloxy - 3,3 - diméthylbutane - 2 - one selon la revendication 1.

4. 1 - (1,2,4 - triazol - 1 - ) - 1 - (4' - phénylazo) - phényloxy - 3,3 - diméthylbutane - 2 - one choisie dans le groupe constitué par les composés suivants:

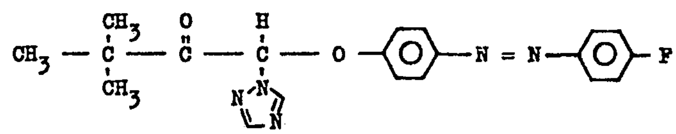

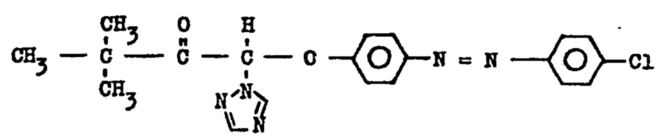

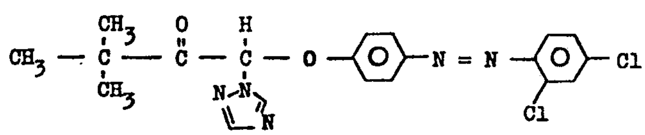

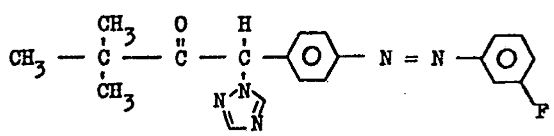

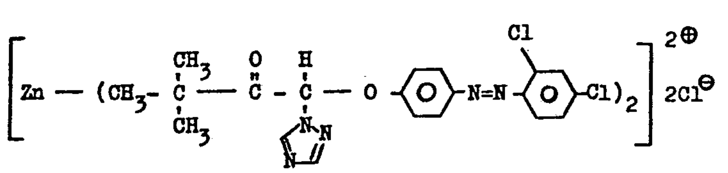

**Claims**

1. 1 - (1,2,4 - triazolyl - (1)) - 1 - (4' - phenylazo) - phenyloxy - 3,3 - dimethylbutan - 2 - one compounds of the general formula I

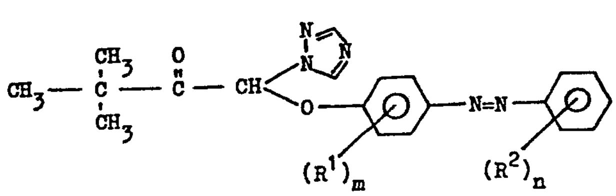

(I)

where $R^1$ and $R^2$ are identical or different and denote alkyl of a maximum of 4 carbon atoms, alkoxy of a maximum of 4 carbon atoms, alkylsulfoxy of a maximum of 4 carbon atoms, halogen, trifluoromethyl or nitro, m denotes one of the integers 0, 1, 2, 3 and 4, and n denotes one of the integers 0, 1, 2, 3, 4 and 5, or their salts or metal complex compounds of the metals Cu, Zn, Sn, Mn, Fe, Co and Ni.

2. A fungicide containing a 1 - (1,2,4 - triazolyl - (1)) - 1 - (4′ - phenylazo) - phenyloxy - 3,3 - dimethylbutan - 2 - one compound as claimed in claim 1.

3. A process for the production of a fungicide, characterized in that a solid or liquid carrier is mixed with a 1 - (1,2,4 - triazolyl - 1) - 1 - (4′ - phenylazo) - phenyloxy - 3,3 - dimethylbutan - 2 - one compound as claimed in claim 1.

4. A 1 - (1,2,4 - triazole - 1) - 1 - (4′ - phenylazo) - phenyloxy - 3,3 - dimethylbutan - 2 - one compound selected from the group consisting of

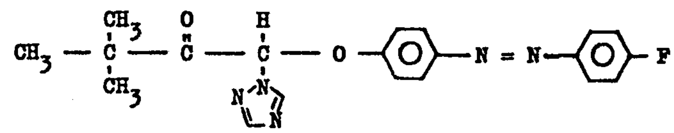

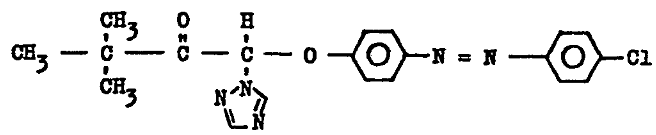

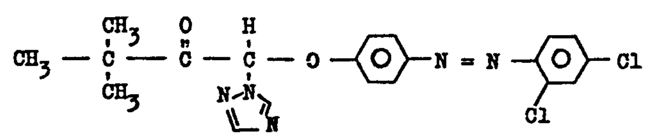

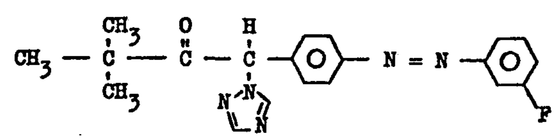

, and